# EUROPEAN PATENT APPLICATION

(11) **EP 4 431 940 A1**
(43) Date of publication of application: **18.09.2024**
(21) Application number: 24153584.8
(22) Date of filing: 24.01.2024
(51) Int. Cl.: G01N 33/00, G01N 27/66

(54) **PHOTOIONIZATION DETECTOR SENSOR WITH ADJUSTABLE GAIN**

(30) Priority: 15.03.2023 US 202318184037
(71) Applicant: Mocon, Inc., Minneapolis, Minnesota 55428 (US)
(72) Inventor: Willcox, Charles, Hopkins, 55343 (US); Jennings, David, Edina, 55439 (US); Fields, Christopher, Coon Rapids, 55433 (US)
(74) Representative: Vossius & Partner Patentanwälte Rechtsanwälte mbB

(57) **Abstract**

A photoionization detector sensor (100) equipped with an adjustable gain amplifier (140), and method of standardizing output of the photoionization detector sensor (100) by adjusting the gain so that an actual test value expected to produce a known anticipated value matches the anticipated value.

## Description

### BACKGROUND

Photoionization detector (PID) sensors break molecules, typically volatile organic molecules, into positively charged ions and free electrons using high-energy ultraviolet (UV) radiation. As target analyte enters the detector, they are bombarded by high-energy UV photons. The target analyte absorbs the UV light, resulting in ejection of electrons and the formation of positively charged ions. The positively charged ions are sensed by an anode/cathode arrangement in the detector and produce an electric current whose amplitude is proportional to the positively charged ions produced (*i.e.*, the greater the concentration of target analyte in the sample entering the detector the more positively charged ions produced resulting in a greater current). The current is amplified, displayed on an ammeter, or converted via a look-up table to a concentration of target molecules and digitally displayed.

Manufacturing variances often result in small but meaningful variances in the output of the PID sensors, even amongst those in the same manufactured batch or lot. This requires customers to perform an initial calibration of the PID sensor after placement of the sensor in the customer's instrument.

Hence, a substantial need exists for an OEM PID sensor that eliminates or compensates for manufacturing variances whereby the output of the OEM PID sensor is consistent from sensor to sensor as delivered to customers so as to eliminate the need for customers to perform an initial calibration of the PID sensor after placement of the sensor in the customer's instrument.

### SUMMARY OF THE INVENTION

A first aspect is a photoionization detector sensor equipped with an adjustable gain amplifier. The photoionization detector sensor includes an ultraviolet lamp, electrodes (*e.g.*, an anode-cathode pair), and a signal amplifier. The ultraviolet lamp is operable for ionizing a target analyte within a sample. The electrodes are operable for detecting the presence of ionized target analyte within the sample and generating a current signal proportional to the concentration of target analyte within the sample. The amplifier is in electrical communication with the electrodes for receiving the generated current signal and amplifying the current signal for creating an amplified electrical signal proportional to the concentration of target analyte within the sample. The amplifier includes an adjustable gain feature for allowing the amplified electrical signal output to be standardized to correct for any manufacturing-imposed variance in the current signal generated by the electrodes and transmitted to the amplifier.

A preferred option for the amplifier is a two-stage transimpedance amplifier having an inverting current to voltage first stage and a non-inverting voltage gain second stage wherein the second stage is equipped with a nonvolatile digital potentiometer operable for adjusting the voltage gain.

The photoionization detector sensor can include a housing defining a sample retention chamber operable for holding the sample, the ultraviolet lamp operable for ionizing target analyte within the sample held within the retention chamber, and the electrodes operable for detecting the presence of ionized target analyte within the sample retention chamber.

A second aspect is a method of standardizing output of the photoionization detector sensor in accordance with the first aspect. The method involves the steps of (A) activating the photoionization detector sensor to detect target analyte in a sample to create an amplified electrical signal having a test value, wherein the sample has a known concentration of the target analyte and is expected to generate an amplified electrical signal of known anticipated value, (B) comparing the test value and the anticipated value, and (C) standardizing output of the photoionization detector sensor by adjusting the gain imposed by the amplifier so that the gain-adjusted test value matches the anticipated value.

### BRIEF DESCRIPTION OF THE DRAWINGS

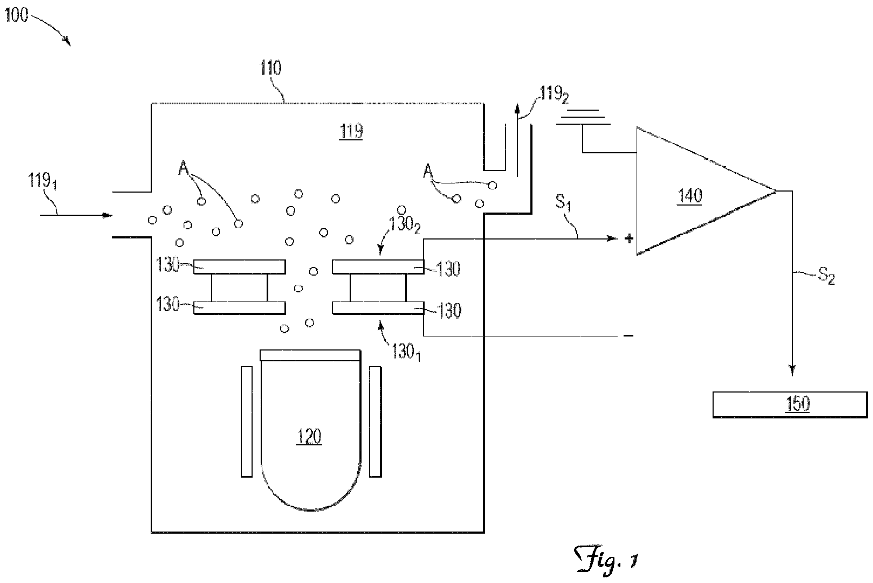

Figure 1 is a schematic view of one embodiment of the invention.

### DETAILED DESCRIPTION OF A PREFERRED EMBODIMENT

### Nomenclature Table

| **REF. NO.** | **DESCRIPTION** |
|---|---|
| **100** | Photoionization Detector Sensor |
| **110** | Housing |
| **119** | Sample Retention Chamber |
| **119₁** | Sample Intake Port |
| **119₂** | Sample Venting Port |
| **120** | UV Lamp |
| **130** | Electrodes |
| **130₁** | First Electrode or Anode |
| **130₂** | Second Electrode or Cathode |
| **140** | Amplifier with Adjustable Gain |
| **150** | Processor |
| **S₁** | Current Signal from Electrodes |
| **S₂** | Amplified and Gain Adjusted Signal from Amplifier |
| **A** | Target Analyte |

### Construction

Referring to Figure 1, the invention is a photoionization detector sensor **100** equipped with an adjustable gain amplifier **140**.

Photoionization detector sensors **100** have an ultraviolet (UV) lamp **120** for ionizing target analyte **A** within a sample, a pair of electrodes **130** (*i.e.*, an anode **130₁** and a cathode **130₂**) for detecting the ions and generating a first electrical current signal **S₁** proportional to the concentration of target analyte **A** within the sample, and an amplifier **140** in electrical communication with the electrodes **130** for receiving the generated first electrical current signal **S₁** and amplifying and converting the first electrical current signal **S₁** to a second voltage electrical signal **S₂**. These components are generally retained within a housing **110** that defines a sample retention chamber **119** positioned to receive UV radiation emitted by the UV lamp **120** upon excitation of the lamp **120** and having a sample intake port **119₁** and optionally a sample venting port **119₂**.

Photoionization detector sensors **100** are employed in instruments that typically include a processor **150** for receiving the amplified electronic signal **S₂**, converting the value of the amplified electronic signal **S₂** to a concentration of target analyte **A** in the sample based upon an algorithm or a lookup table, and displaying or otherwise reporting the concentration.

The photoionization detector sensor **100** of the present invention has an adjustable gain amplifier **140**, such as a two-stage transimpedance amplifier with an inverting current to voltage first stage and a non-inverting voltage gain second stage, wherein the second stage is equipped with a nonvolatile digital potentiometer operable for adjusting the voltage gain.

The photoionization detector sensor **100** is particularly adapted to detect and quantify the concentration of volatile organic compounds in a sample.

### Factory Standardization

The method of standardizing output of the photoionization detector sensor **100** includes the steps of (A) activating the photoionization detector sensor **100** to detect target analyte **A** in a sample to create an amplified electrical signal **S₂** having a test value, wherein the sample has a known concentration of the target analyte **A** and is expected to generate an amplified electrical signal of known anticipated value, (B) comparing the test value and the anticipated value, and (C) standardizing output of the photoionization detector sensor **100** by adjusting the gain imposed by the amplifier **140** so that the gain-adjusted test value matches the anticipated value.

## Claims

1. A photoionization detector sensor (100) equipped with an adjustable gain amplifier (140).

2. A photoionization detector sensor (100) having (i) an ultraviolet lamp (120) operable for ionizing a target analyte (A) within a sample, (ii) electrodes (130) for detecting the presence of ionized target analyte (A) within the sample and generating a current signal (S₁) proportional to the concentration of target analyte (A) within the sample, and (iii) an amplifier (140) in electrical communication with the electrodes (130) for receiving the generated current signal (S₁) and amplifying the current signal (S₁) via an adjustable gain to create an amplified electrical signal (S₂) proportional to the concentration of target analyte (A) within the sample.

3. The photoionization detector sensor (100) of claim 2 wherein the amplifier (140) is a two-stage transimpedance amplifier having an inverting current to voltage first stage and a non-inverting voltage gain second stage wherein the second stage is equipped with a nonvolatile digital potentiometer operable for adjusting the voltage gain.

4. The photoionization detector sensor (100) of claim 2 or 3, wherein the ultraviolet lamp (120) is operable for ionizing a volatile organic compound target analyte (A).

5. A photoionization detector sensor (100) having (i) a housing (110) defining a sample retention chamber (119), (ii) an ultraviolet lamp (120) operable for ionizing a target analyte (A) within the sample retention chamber (119), (iii) an anode-cathode pair (130₁ and 130₂) for detecting the presence of ionized target analyte (A) within the sample retention chamber (119) and generating a current (S₁) proportional to the concentration of target analyte (A) within the sample retention chamber (119), and (iv) a two-stage transimpedance amplifier (140) in electrical communication with the anode-cathode pair (130₁ and 130₂) for receiving the generated current (S₁) and having an inverting current to voltage first stage to create an electrical signal having a voltage proportional to the concentration of target analyte (A) within the sample, and a non-inverting voltage gain second stage equipped with a nonvolatile digital potentiometer operable for adjusting the voltage gain to create an amplified and adjusted electrical signal (S₂) proportional to the concentration of target analyte (A) within the sample.

6. The photoionization detector sensor (100) of claim 5 wherein the ultraviolet lamp (120) is operable for ionizing a volatile organic compound target analyte (A).

7. A method of standardizing output of a photoionization detector sensor (100) in accordance with any one of claims 2 to 4, comprising the steps of (A) activating the photoionization detector sensor (100) to detect target analyte (A) in a sample to create an amplified electrical signal (S₂) having a test value, wherein the sample has a known concentration of the target analyte (A) and is expected to generate an amplified electrical signal (S₂) of known anticipated value, (B) comparing the test value and the anticipated value, and (C) standardizing output of the photoionization detector sensor (100) by adjusting the gain imposed by the amplifier (140) so that the gain-adjusted test value matches the anticipated value.

8. A method of standardizing output of a photoionization detector sensor (100) in accordance with claim 5 or 6, comprising the steps of (A) activating the photoionization detector sensor (100) to detect target analyte (A) in a sample to create an amplified electrical signal (S₂) having a test value, wherein the sample has a known concentration of the target analyte (A) and is expected to generate an amplified electrical signal (S₂) of known anticipated value, (B) comparing the test value and the anticipated value, and (C) standardizing output of the photoionization detector sensor (100) by adjusting the gain imposed by the non-inverting voltage gain second stage of the two-stage transimpedance amplifier (140) so that the gain-adjusted test value matches the anticipated value.
